# EUROPEAN PATENT SPECIFICATION

(11) **EP 4 526 642 B1**
(45) Date of publication and mention of the grant of the patent: **31.12.2025**
(21) Application number: 23726527.7
(22) Date of filing: 17.05.2023
(51) Int. Cl.: G01N 1/34, G01N 1/40

(54) **MULTI-BED TRAP FOR WATER ISOTOPE ANALYSIS**
MEHRBETTFALLE ZUR WASSERISOTOPENANALYSE
PIÈGE À LITS MULTIPLES POUR ANALYSE ISOTOPIQUE DE L'EAU

(30) Priority: 17.05.2022 LU 502099
(43) Date of publication of application: 26.03.2025
(73) Proprietor: LUXEMBOURG INSTITUTE OF SCIENCE AND TECHNOLOGY (LIST), 4362 Esch-sur-Alzette (LU)
(72) Inventor: LOPEZ DIAS, Veneranda, 4362 Esch-sur-Alzette (LU); BARNICH, François, 4362 Esch-sur-Alzette (LU); SCHOPPACH, Rémy, 4362 Esch-sur-Alzette (LU)
(74) Representative: Ipsilon Luxembourg
(86) International application number: PCT/EP2023/063346
(87) International publication number: WO 2023/222806

(56) References cited:
- WO-A1-2021/056943
- CN-A- 106 198 405
- STEINBACHER M ET AL: "Performance characteristics of a proton-transfer-reaction mass spectrometer (PTR-MS) derived from laboratory and field measurements", INTERNATIONAL JOURNAL OF MASS SPECTROMETRY, ELSEVIER SCIENCE PUBLISHERS , AMSTERDAM, NL, vol. 239, no. 2-3, 15 December 2004 (2004-12-15), pages 117 - 128, XP004676183, ISSN: 1387-3806, DOI: 10.1016/J.IJMS.2004.07.015
- DOLORES ASENSIO ET AL: "On-line screening of soil VOCs exchange responses to moisture, temperature and root presence", PLANT AND SOIL ; AN INTERNATIONAL JOURNAL ON PLANT-SOIL RELATIONSHIPS, KLUWER ACADEMIC PUBLISHERS, DO, vol. 291, no. 1-2, 23 January 2007 (2007-01-23), pages 249 - 261, XP019507471, ISSN: 1573-5036, DOI: 10.1007/S11104-006-9190-4

## Description

### FIELD OF THE INVENTION

The invention relates to systems and methods for purifying water samples prior to performing a water isotope analysis without fractionating the original isotope composition.

### BACKGROUND ART

The isotope-ratio infrared spectroscopy (IRIS) is the technique typically used to measure the isotope ratios of oxygen and hydrogen in water. The two instruments available in the market are Los Gatos Research (LGR), an off-axis integrated cavity output spectroscopy (OA-ICOS) analyser and Picarro Inc, a wavelength-scanned cavity ring-down spectroscopy (WS-CRDS) analyser.

Some contaminants typically co-distilled during the cryogenic extraction of water from plants or soils, such as ethanol (EtOH), methanol (MeOH) and formic acid, lead to erroneous reading by these instruments, as the absorbance of these compounds is greater than the absorbance of water (H₂O) and of semi-heavy water (HDO). This drawback is also occurring in vapour equilibration technique, since they are volatile organic compounds. Other organic compounds also act as interfering absorbers (higher molecular weight alcohols, CH₄, H₂O₂...). Additionally, impure water samples reduce the performance of the analyser (i.e. memory effects, clogged filters, sample cell integrity).

Various techniques have been developed for overcoming the issue of contaminants when attempting to analyse water stable isotopes, either by correcting the results obtained by the measuring instrument to take the presence of contaminants under consideration, or by attempting to clean the water before their analysis. However, these known techniques have the following drawbacks.

LGR uses a post-processing software to identify and quantify the spectral contamination and to correct the isotopic ratio of contaminated samples (Schultz et al., "Identification and correction of spectral contamination in 2H/1H and 18O/16O measured in leaf, stem and soil water", DOI 10.1002/rcm.5236). This software operates as a function of the degree of contaminant concentration and of the type of contaminant, and hence each experiment and each analyser require a customized correction. Also, it appears that this solution does not enable to correct the δ²H value for EtOH contamination and it is not suitable for samples with unknown composition. Martín-Gómez (Martín-Gómez et al., "Isotope-ratio infrared spectroscopy: a reliable tool for the investigation of plan-water sources?", DOI 10.1111/nph.13376) discusses a post-processing software used by Picarro Inc. (ChemCorrect^{™}) that is based on peaks filtering. Although this software seems to be more suitable than the correction based on contaminant concentration, it does not work for EtOH contamination. Moreover, in the actual version ChemCorrect is only due to flag the samples where the analysis was biased by spectral interference.

Also, Picarro proposes a Micro-Combustion Module (MCM) to remove the organic matter by high temperature oxidation. The main issue with MCM is that the combustion generates exogenous water in the samples, since the products of the oxidation of organics are CO₂ and H₂O. This exogenous water comes from the organic matter present in the sample and from the exogenous oxygen introduced in the system to carry out the oxidation. This exogenous water could create few per mil offset in δ²H and δ¹⁸O values even at low contamination levels in water samples (Leen et al., "Spectral contaminant identifier for off-axis integrated cavity output spectroscopy measurements of liquid water isotopes", DOI 10.1063/1.4704843; Martín-Gómez et al., "Isotope-ratio infrared spectroscopy: a reliable tool for the investigation of plant-water sources?", DOI 10.1111/nph.13376). Moreover, primary alcohol oxidation could occur instead of complete combustion, generating some aldehydes and formic acid in the post-MCM water (Chang et al., "Improved removal of volatile organic compounds for laser-based spectroscopy of water isotopes", DOI 10.1002/rcm.7497), both compounds causing spectral interferences. Chang et al. suggest that the creation of formic acid would explain the worse results obtained for EtOH contaminated water when using the MCM.

Aside from MCM, other pre-treatment techniques have been used to remove the organic matter before the introduction of the sample into the analysis instrument.

For instance, activated charcoals have been used to trap and bind the ethyl and methyl groups of EtOH and MeOH in water. Despite the fact that this performs well to remove high molecular weight organics from water samples, the adsorption of EtOH and MeOH was not found to be significant enough to completely eliminate the spectral interferences (see Shultz *et al.,* Chang *et al*.).

Also, a Solid-Phase Extraction technique (SPE) has been investigated to filter water prior to analyses. This technique makes specifically use of an octadecyl (C-18) adsorbent bonded to porous silica (Chang *et al*.). This adsorbent adsorbs the ethyl and methyl groups (non-polar) of EtOH and MeOH and lets the H₂O (polar) pass through. Despite this SPE technique being more efficient than the activated charcoal, it is still not performing well enough on MeOH contaminated waters. Chang *et al.* have proposed an automated in-line system that incorporates SPE upstream of MCM to reduce the quantity of alcohols in the (liquid) water before it reaches the MCM, thereby reducing the amount of exogenous water generated during the combustion of the remaining organic matter. This system has proven inefficient for MeOH contaminated waters. The technique has a recovery yield limited to 90% and a suitable volume of 0.5 ml, it is time-consuming and has not been tested for the combination of several alcohols and other organics.

WO2021/056943A1 and CN106198405B disclose prior art systems and methods for purifying water vapour samples.

### SUMMARY OF THE INVENTION

The invention aims at offering a system and a method for water stable isotope analysis that overcomes the limitations of previous techniques and is more reliable and simpler than known techniques.

The invention relates to a system for purifying water vapour samples prior to water stable isotope analysis, characterized by: a pipe with a multi-bed trap that comprises at least one hydrophobic graphitized black carbon sorbent, GBC and a hydrophobic carbon molecular sieve sorbent, CMS; an analyser for analysing the isotope ratio of the purified water vapour; and a pump operatively connected to the pipe and optionally constituting a part of the analyser, to lead water vapour through the at least one hydrophobic graphitized black carbon sorbent and then through the hydrophobic carbon molecular sieve sorbent. The (combination of) GBC adsorbent(s) improves the efficiency of the CMS to trap EtOH and MeOH. This overall system is versatile as it is adapted to trap a wide variety of organic compound families and molecular weights without altering the original isotope composition of the water sample. The system is thus simple and reliable. The pump may be a separated entity or may be integrated into the analyser.

The wording "purifying" is to be understood in connection with the above discussion in relation to the elements which perturb the measurements of isotope ratio analysis.

According to a preferred embodiment, the at least one hydrophobic graphitized black carbon sorbent comprises a first sorbent and a second sorbent, the water vapour passing through the first sorbent before passing through the second sorbent, the first sorbent having a lower effective surface area than the second sorbent.

Thus, the first sorbent retains high molecular weight organic compounds (relative analytical size C₁₂-C₂₀) and the second sorbent retains mid-molecular weight organic compounds (relative analytical size C₅-C₁₂). This arrangement of sorbents facilitates the purification of the contaminated water samples, improving the adsorption efficiency of the CMS bed placed at the end of the trap for EtOH and MeOH.

According to a preferred embodiment, the effective surface area of the first sorbent is lower than 25 m²/g and/or the effective surface area of the second sorbent is greater than 90 m²/g.

According to a preferred embodiment, the density of the first sorbent GBC is greater (0.68 g/mL) than the density of the second GBC sorbent (0.35 g/mL) and the density of the CMS is 0.61 g/mL.

According to a preferred embodiment, the GBC sorbent(s) and the CMS sorbent have a mesh size of 60/80 or 40/60. This enables to avoid channelling (smaller mesh size) and excessive back pressure (larger mesh size). The effective surface area of the first GBC sorbent (10 m²/g) is lower than the effective surface area of the second GBC sorbent (100 m²/g), and the CMS has a greater surface area (975 m²/g). The higher the effective surface area value, the stronger the sorbent. The water vapour passes from the weakest sorbent towards the strongest sorbent. The high molecular weight organic compounds are trapped in the first GBC sorbent, the mid-molecular weight organic compounds are trapped in the second GBC and the low molecular weight compounds are trapped in the CMS sorbent.

According to a preferred embodiment, the effective surface area of the hydrophobic carbon molecular sieve sorbent (18) is greater than 800 m²/g. The carbon molecular sieve sorbent is thus stronger than the graphitized black carbon sorbents, the carbon molecular sieve sorbent retaining the low molecular weight organic compounds (relative analytical size C₂-C₅), including MeOH.

According to a preferred embodiment, the system comprises a third hydrophobic graphitized carbon black sorbent placed upstream the first hydrophobic graphitized carbon black sorbent, the third hydrophobic graphitized carbon black sorbent having an effective surface area lower than the effective surface of the two subsequent hydrophobic graphitized carbon black sorbents. The third sorbent can retain high molecular weight organic compounds (relative analytical size > C₂₀).

According to a preferred embodiment, the effective surface area of the third hydrophobic graphitized carbon black sorbent is lower than 10 m²/g.

According to a preferred embodiment, the system comprises at least one inert and hydrophobic frit filter interposed between the sorbents and/or retaining the sorbents in the pipe.

According to a preferred embodiment, the at least one inert and hydrophobic frit filter is an inert-coated stainless steel frit filter or a quartz frit filter or a glass frit filter.

According to a preferred embodiment, the system further comprises a hydrophobic retaining element arranged downstream of, and to retain, the hydrophobic frit filter, the hydrophobic retaining element preferably being formed by an inert-coated stainless steel wire cloth basket.

According to a preferred embodiment, the pipe has an internal diameter of about 4 mm; and/or the multi-bed trap spans over about 60 mm of length of the pipe. These dimensions are appropriate for the particular applications of the invention.

According to a preferred embodiment, the system comprises a vapour source.

According to a preferred embodiment, the vapour source is a sublimation system connected to the pipe and forming water vapour by sublimation from a solid sample.

According to a preferred embodiment, the vapour source is a cryo-extraction system; or a transfer system connected to the pipe and forming water vapour by sublimation from a solid sample or by transfer from a collection system.

According to a preferred embodiment, the vapour source is an inlet system connected to the pipe, the inlet system being configurated to directly introduce the sample in vapour phase (vapour mode) towards the pipe or the inlet system being a combination of injector and vaporizer (liquid mode) to vaporize the injected liquid water sample before being directed to the pipe.

According to a preferred embodiment, the pipe comprises at least one U-shape cold trap arranged upstream and/or downstream of the multi-bed trap.

According to a preferred embodiment, the analyser is directly connected to the cold trap placed downstream the multi-bed trap.

According to a preferred embodiment, the pipe comprises at least one sample tube arranged upstream and/or downstream of the multi-bed trap.

According to a preferred embodiment, the analyser is directly connected to the multi-bed trap.

According to a preferred embodiment, the analyser is an isotope-ratio infrared spectroscopy, IRIS, based instrument, i.e., a wavelength-scanned cavity ring-down spectroscopy analyser, or a mass spectrometer.

The invention also relates to a method for purifying and analysing water vapour comprising: leading water vapour in a pipe through at least one hydrophobic graphitized black carbon sorbent and then through a hydrophobic carbon molecular sieve sorbent; and then analysing the isotope ratio of the purified water vapour.

According to a preferred embodiment, the method is carried out in a system as mentioned above.

The various aspects of the invention mentioned above present many benefits: the methodology enables to purify contaminated water samples from a wide range of organic compounds and a wide range of molecular weights. Hence the application area is wide, as it can be applied to the analyses of waters extracted from different matrices. This versatility simplifies the cleaning process because it renders unnecessary the use of multiple specific pre-treatment methodologies for each contaminant and the creation of artefacts and/or exogenous water during the reactions carried out for purification.

Additionally, a post-processing software is not required and the method is therefore more reliable as there is no more errors inherent to post-processing (depending on the concentration of contaminants in water samples and the spectral interferences for each peak).

Also, the multi-bed trap can be used in line, since it can be arranged directly between the injector and the measuring instrument. The multi-bed trap can be easily cleaned by an inverted flow of nitrogen or helium. This cleaning procedure will desorb the organic compounds in a direction opposite to their adsorption. An organic trap waste container can be used for collecting these compounds.

Moreover, the method and system of the invention can be applied to both the cryogenic extraction and the vapour equilibration methodologies, currently used for water extraction from soils and plants, as well as to sublimation system or in combination with IRIS or IRMS instruments. Despite the fact that the vapour equilibration is a simpler, faster and less expensive process (Orlowski et al., Intercomparison of soil pore water extraction methods for stable isotope analysis, DOI 10.1002/hyp.10870), it is more affected by the volatile organic contaminants (Millar et al., A Comparison of Extraction Systems for Plant Water Stable Isotope Analysis, DOI 10.1002/rcm.8136). In this context, the multi-bed trap may be relevant to remove organic contaminants and automatize the vapour equilibration technique.

Finally, the versatility of the method and system of the invention enables a wide range of areas of application: the study of the isotopic composition of water is relevant for a wide range of research fields, such as paleoclimatology (ice core water isotopes, cave ice water isotopes, temporal (18O/16O)/temperature relationship, plants water composition), environmental monitoring (groundwater system water quality, parasitic discharge in sewers, contamination origin), ecology (plant water uptake, water isotopes as tracers of diet and provenance in the biota of an aquatic ecosystem), forensics (geographic origin of plant fibre present on clothes, geographic sourcing of wine, dietary and water source information recorded in hair and fingernails to distinguish individuals of different geographic origin, possible sources of mad cow disease, vectors associated with the bird flu, food authenticity) and hydrology (water sources, pathways and transit times). The multi-bed trap can be used for *in situ* analyses for instance for (contaminated) waters from rivers or wastewater.

### BRIEF DESCRIPTION OF DRAWINGS

Figure 1 shows a multi-bed trap;
Figures 2 to 6 illustrate various implementations of the multi-bed trap;
Figure 7 shows results of a first experiment;
Figure 8 shows a result from a second experiment.

### DETAILED DESCRIPTION OF EMBODIMENTS OF THE INVENTION

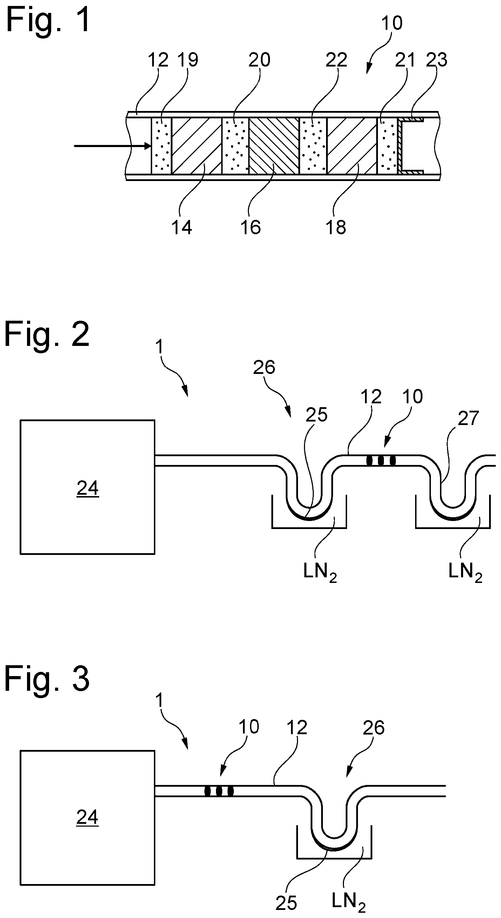

Figure 1 shows an example of a multi-bed trap 10. A pipe segment 12 is occupied by two hydrophobic graphitized black carbon sorbents (GBC) 14, 16 and by a hydrophobic carbon molecular sieve (CMS) sorbent 18. Obviously, the person skilled in the art would understand that the number of GBCs can be varied. The CMS 18 could alternatively be used alone (without GBC).

The first GBC 14 can have an effective surface area lower than 25 m²/g. An example is the commercially available Carbopack^{™} C.

The second GBC 16 can have an effective surface area greater than 90 m²/g. An example is the commercially available Carbopack^{™} B.

The CMS 18 can be a carbon molecular sieve sorbent having an effective surface area greater than 800 m²/g. An example is the commercially available Carbosieve^{™} SIII.

The three adsorbents are hydrophobic and thus they let water freely move through without being trapped and without altering its isotope signature. The GBCs retain the mid- to large molecular weight compounds, while the CMS retains the low molecular weight compounds including MeOH. More specifically, the first GBC with lower surface area traps the high molecular weight organic compounds (relative analytical size C₁₂-C₂₀), the second GBC with greater specific surface area traps mid-molecular weight organic compounds (relative analytical size C₅-C₁₂), and the CMS with the greatest specific surface area traps low molecular weight organic compounds including methanol (relative analytical size C₂-C₅). If the target samples contain organic compounds with a relative analytical size > C₂₀, other extra GBC with specific surface area lower than 10 m²/g should be added as first sorbent (i.e., Carbopack^{™} F, with specific surface area 5 m²/g).

The water vapour that reaches the CMS is already partially cleaned by the GBCs, leading to a less frequent need to clean the trap. This arrangement also improves the adsorption efficiency of the CMS placed at the end of the trap for EtOH and MeOH, as well as the overall trapping efficiency. Adsorbing firstly the higher molecular weight organics allows a better absorption of the lower molecular weight organics: if the higher molecular weight organics were not adsorbed first, they would form nets through which the lower molecular weight organics could pass without being adsorbed (as they would not get closer to the sorbent).

Two separators 20, 22 for instance made of inert-coated stainless steel frit filter delimit the sorbents from each other avoiding a mixing of two sorbents. Such a mixture would indeed decrease the retention efficiency. The inert coating prevents the separator from absorbing water when passing through, maximizing the recovering yield and avoiding isotope fractionation of the water sample. The separators are also inert to the water contaminants avoiding secondary reactions. The contaminants can be analysed in a TDU-GC-MS later if needed.

An inert-coated stainless steel frit filter 21 may be provided to retain by an inert-coated stainless steel wire cloth basket 23 retains the sorbents in the outlet of the pipe.

A glass frit filter 19 can retain the sorbent in the inlet of the pipe.

The arrow shows the direction of the flow of water vapour.

Figure 2 illustrates an example of a system 1 according to the invention. The system may comprise a sublimation system 24 configured to sublimate a solid sample containing water. The sublimation system 24 can contain part or all of the system discussed in patent application PCT/EP2021/082753. A solid sample may be sublimated with the assistance of a thermal management system as presented in patent application PCT/EP2021/082789. The sublimation system 24 is connected to a pipe 12 and the vapour generated by the sublimation system is directed into the pipe 12 and forced through the multi-bed trap 10, by a pump (not shown) or by the pump of the analysing instrument (in-line) with which the water isotope analyses will be carried out (i.e. laser spectrometer or mass spectrometer). After water purification, the water stable isotopes would be directly analyzed by the instrument and the trapped organic compounds could be later analyzed by a TDU-GC-MS.

A cold trap 26 can be arranged between the sublimation system 24 and the multi-bed trap 10. The cold trap 26 is essentially a U-shape portion of the pipe 12 that is dipped in a bath of liquid nitrogen as described in the patent application PCT/EP2021/082753. The sublimated compounds are first trapped in the cold trap 26, the cold trap 26 being subsequently heated with a heating element 25. The trapped compounds generated by the sublimation system are directed into the pipe 12 and forced to pass through the multi-bed trap 10, by a pump (not shown) or by the instrument with which the water isotope analyses will be carried out (in-line) (i.e. laser spectrometer or mass spectrometer). The organic compounds are trapped into the multi-bed trap for further analyses with the TDU-GC-MS and the purified water analysed directly by the instrument (in-line) or collected in the second U-shape tube 27 (second cold trap) (off-line), for subsequent transfer into a vial, potentially using the transfer system discussed in the patent application PCT/EP2021/082753 and further isotope analyses.

As shown in the alternative embodiment of figure 3, the cold trap 26 can also be positioned downstream of the multi-bed trap 10. The multi-bed trap (10) purifies the water directly during sublimation before reaching the instrument (in-line) or being collected in the cold trap 26 (off-line) for subsequent transfer into a vial, potentially using the transfer system discussed in the patent application PCT/EP2021/082753 and further isotope analyses. Alternatively, an in-line version could be configured after the cold trap 26 as discussed in the embodiment of figure 2.

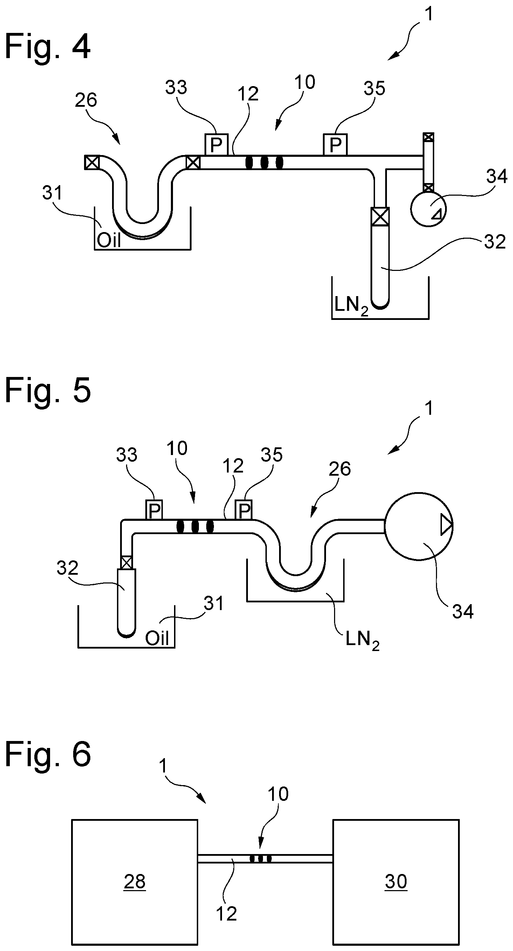

As shown in the alternative embodiment of figure 4, the multibed trap can be placed in the transfer system (described in the patent application PCT/EP2021/082753) when sublimation requires a high evacuation rate. It can be operated as follows: The cold trap 26 containing the sublimating compounds is connected to the transfer system in vacuum. The pump 34 creates a primary vacuum in the rest of the system. The cold trap 26 is heated with a heating element 31 (i.e. oil bath) and the sample tube 32 submerged into LN2. The sublimated compounds are transferred from the U-shape tube 26 to the sample tube 32 in static vacuum by the gradient in pressure. The compounds pass through the multi-bed trap 10 placed in the pipe 12 between the U-shape tube 26 and the sample tube 32. The organic compounds are trapped in the multi-bed trap 10 for further analyses and the purified water is deposited in the sample tube 32 in solid state for further analyses. Alternatively, an analyser can be connected downstream the multi-bed (in-line) trap instead of the sample tube 32 (off-line), the compounds being directed towards the analyser under dynamic vacuum by the pump of the analyser.

Two pressure sensors 33, 35 can be placed upstream and downstream the multi-bed trap to assist monitoring the transfer.

Figure 5 shows a simplified scheme for a set-up for a cryo-extraction experiment. A sample tube 32 containing the frozen sample from which water will sublimate is connected to a cold trap 26. The multi-bed trap 10 is placed into a pipe 12 between the sample tube 32 and the cold trap 26. A pump 34 creates a primary vacuum in the system. The sample tube 32 is heated with a heating element 31 (i.e. oil bath) and the cold trap 26 is submerged into a LN2 bath. Sublimation is carried out under dynamic vacuum and the pump 34 directs the sublimated compounds from the sample tube 32 to the cold trap 26 (off-line) passing through the multi-bed trap 10. In the cold trap 26, the purified sublimated water will deposit (it does not reach the pump 34). The organic compounds will be retained in the multi-bed trap. Pressure sensors and valves can be arranged along the pipe 12 to control the experiment. As an example, two pressure sensors 33, 35 can be placed upstream and downstream of the multi-bed trap.

Figure 6 shows another embodiment of the system 1 of the invention. An inlet 28 is foreseen to introduce the sample in vapour phase into the pipe 12. The vapour passes through the multi-bed trap 10 before reaching an analyser 30. The analyser 30 may be an isotope-ratio infrared spectroscopy (IRIS) based instrument (i.e, wavelength-scanned cavity ring-down spectroscopy analyser) or other analyser (i.e, a mass spectrometer). In any case, the invention enables both laboratory and *in situ* analyses. The purified water will be analysed by the analyser and the organic compounds trapped by the multi-bed trap. The organic compounds could be analysed later with a TDU-GC-MS.

The inlet 28 may be a system comprising an injector and a vaporizer (liquid mode). The system vaporizes the injected water sample and directs the vapour into the pipe 12 through the multi-bed trap. Alternatively, the inlet 28 may be a system that introduces directly the sample when the water sample is already in its vapour phase (vapour mode).

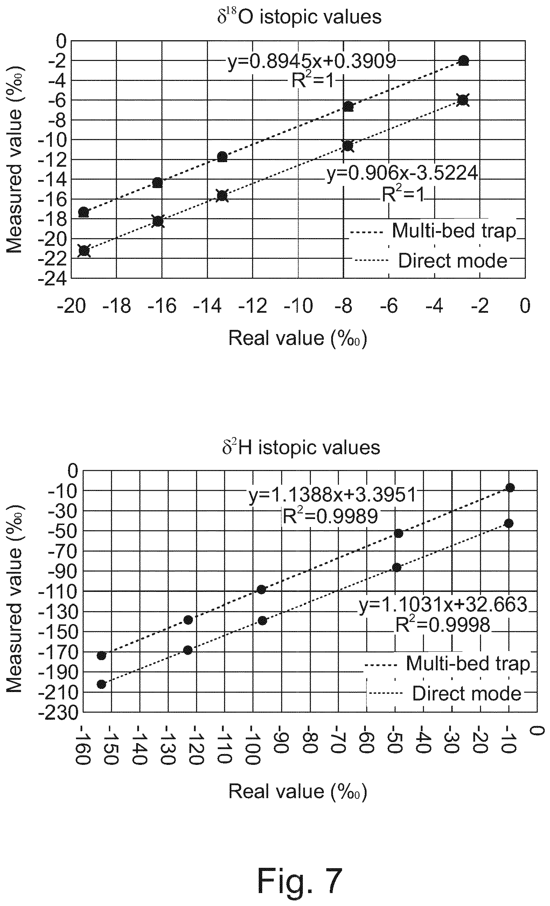

Figure 7 shows the results of a first experiment done with the embodiment described in figure 6 for the vapour mode. This experiment aims at demonstrating that the multi-bed trap 10 does not alter the initial isotope signature of the water sample, by comparing the vapour purified by the multi-bed trap 10 originated from standard samples and the vapour originated from standard samples introduced directly in the analyser without passing through the multi-bed trap 10.

The multi-bed trap used here corresponds to the one discussed above. In comparison to Figure 1, the separators (between the sorbents) were here quartz wool and the retainer in the outlet of the multi-bed trap was quartz wool as well.

Five water standards samples from Los Gatos Research (LGR): 1C, 2C, 3C, **4E,** and 5C were used. The isotopic values of the water standards relative to the Vienna Standard Mean Ocean Water (VSMOW) are -154 ± 0.5 ‰, -123.7 ± 0.5 ‰, -97.3 ± 0.5 ‰, -49.2 ± 0.5 ‰ and -9.2 ± 0.5 ‰, respectively, for δ²H and -19.57 ± 0.1 ‰, -16.24 ± 0.1 ‰, -13.39 ± 0.1 ‰, -7.81 ± 0.1 ‰ and -2.69 ± 0.15 ‰ for δ¹⁸O. 1 ml of each standard was placed into a bag of 2 l capacity. The 5 bags were filled with a mixture of gas 80 % N₂ and 20 % O₂ quality alphagaz α1 (from company Air Liquide) and kept at 23.5 °C over the night (19 hours) to allow the water vapour to saturate the gas. Then, the water vapour from the five standards were measured with a PICARRO cavity ring down laser spectrometer L2140-i. A comparison was made between directly measured water isotopic ratios of these standards, and water isotopic ratios measured after the vapour passed through the multi-bed trap 10.

The concentration of water vapour in the bags is 30000 ppmv and the flow to the spectrometer 40 sccm. The water vapour was analysed continuously during 9 min and then the spectrometer was flushed with dry air for 6 min. It was flushed for 20 min for the experiments using the multi-bed trap to ensure the elimination of the remaining water in the multi-bed trap.

The amount of water going through the trap is 7.89 mg and the water remaining in the trap is 2.4 mg. Thus, 30.4 % of the water vapour is retained by the trap, most of it absorbed by the quartz wool between the hydrophobic sorbents. The measured isotopic ratio for the water vapour is converted into the liquid water value and the results are shown in figure 7.

The isotopic values for both the δ¹⁸O and the δ²H are more depleted after passing through the multi-bed trap. Nevertheless, the calibrates are linear both the direct measurement and the indirect measurement (after going through the multi-bed trap) have similar slop with different offset. The coefficient of regression R2 is good for both direct and indirect measurements and for both the δ¹⁸O and δ²H isotopic values. Despite the fact that the use of multi-bed trap results in more depleted values for the analysed water, these graphs show that the behaviour is reproducible and that the calibrate is linear. Hence, the deviation between indirect measurement and direct measurement could be corrected with the calibrate. Nevertheless, a modification was done in the multi-bed trap to avoid trapping water and to maximize the recovery yield on one side while avoiding any modification in the original isotope signature on the other side. For this purpose, the quartz wool was substituted by inert-coated stainless steel frit filters as shown in Fig. 1.

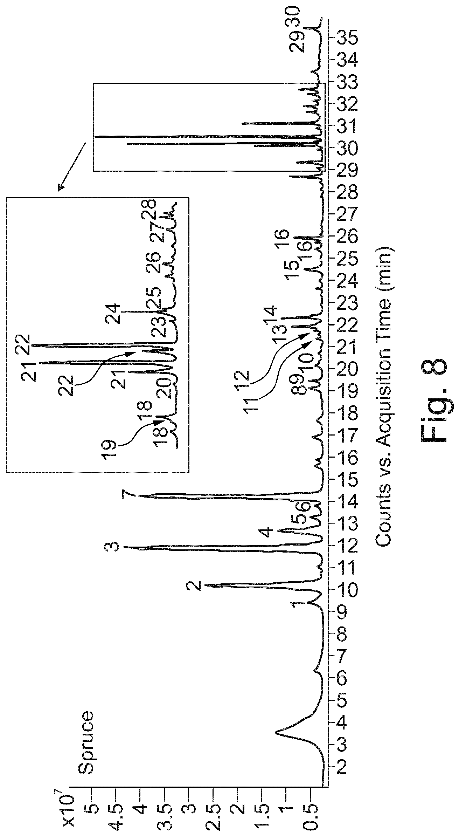

Figure 8 shows the results of a second experiment with the set-up described in figure 5 for cryo-extraction, done to demonstrate the capacity of the multi-bed trap to retain different organics. A core of spruce wood was used as sample for the experiment, since it contains naturally a mixture of organics previously identified by GC-MS (Wajs et al., Characterisation of volatile organic compounds in stem wood using solid-phase microextraction, DOI 10.1002/pca.891) and water.

The multi-bed trap that was used corresponds to the one shown on figure 1 and discussed above, with the difference that before the separators between sorbents were quartz wool and the retainer in the outlet was also quartz wool.

A wood core of spruce (Picea abies) was taken 48 h before the experiment and frozen at -20°C for 24 h. The frozen cylinder of spruce was of about 5 mm in diameter and 7 cm in length. It was dipped into LN2 for 40 minutes before the experiment. The system shown in figure 5 was pumped out, flushed with dry air (80 % N₂/20 % O₂), heated with a heating belt for 10 min and cooled down with an empty glass tube in the place of the multi-bed trap to avoid memory effects. The pressure before and after the glass tube was 5*10⁻³ and 10⁻³ mbar respectively. Then the glass tube was substituted by the multi-bed trap and the frozen cylinder of spruce was introduced in the sample tube 32.

Before creating the vacuum again in the system, the tube with the frozen wood was introduced into a LN2 bath to avoid the loss of some volatiles. The system was pumped out reaching a pressure of 1.7*10⁻² mbar upstream of multi-bed trap and 10⁻³ mbar downstream of multi-bed trap. The U-shape cold trap 26 was introduced into LN2 to recover the water from the wood core. The sample tube 32 was heated to 82 °C with a heating element 31 (heating belt) and a thermocouple was used to control the temperature. The pressures were constant during the sublimation process, i.e., 7.5*10⁻¹ mbar upstream and 1.8*10⁻¹ mbar downstream. The water vapour was collected in the U-shape cold trap 26 after passing through the multi-bed trap 10. After 2 h and 15 min the pressure dropped to 3.5*10⁻¹ mbar upstream of multi-bed trap and to 1*10⁻³ mbar downstream of the multi-bed trap, indicating the end of the sublimation process. The mass loss of the spruce wood core during sublimation was 659.8 mg and the recovered water was 603 mg (91.39 %). The remaining 8.61% weight loss corresponds to the compounds trapped in the multi-bed trap plus the water trapped in the quartz wool (separators in the old version of the multi-bed trap). The new version of the multi-bed trap as shown in Fig. 1, having as separators the inert coated stain steel frit filters, retains less than the 1% of the extracted water.

In order to check the trapped compounds in the multi-bed trap after sublimation from the spruce wood core the multi-bed trap was introduced in the thermal desorption unit (TDU) of the GC-MS. The multi-bed trap was flushed with a 40 sccm flow of He gas for 10 seconds to remove the trapped oxygen and water. Then the organic compounds were desorbed by heating at a rate of 120 °C/min to 300 °C, using He as carrier gas, followed by an isothermal period of 20 min at 300 °C. The compounds were introduced in the GC-MS at 250°C (split 20:1). The column was a capillary HP-5 column. The oven program started at 40 °C (2 minutes) and the temperature was increased at a rate of 3 °C/min to 120 °C, then at 20 °C/min to 250 °C (5 minutes isothermal period). The compounds were identified based on the NIST library within the CG-MS software and the retention time found in the literature (Wajs *et al*.).

Figure 7 shows the chromatogram of the trapped compounds after sublimation from the spruce wood core. The compounds are listed in the Table 1 below.

**Table 1: Volatile organic compounds sublimated from a spruce wood core**

| **Peak number** | **Compound** | **Peak number** | **Compound** |
|---|---|---|---|
| **Monoterpenes** | | 17 | α -Terpenyl acetate |
| 1 | α-Thujene | **Sesquiterpenes** | |
| 2 | α -Pinene | 18 | Longicyclene |
| 3 | β-Pinene | 19 | Cyclosativene |
| 4 | β -Myrcene | 20 | Sativene |
| 5 | α -Phellandrene | 21 | Longifolene |
| 6 | α -Terpinene | 22 | (Z)-Caryophyllene |
| 7 | β -Phellandrene | 23 | Acetone anil* |
| **Functionalized monoterpenoids** | | 24 | (E)-9-epi-Caryophyllene |
| 8 | Pinocarveol | 25 | Farnesene |
| 9 | Verbenol | 26 | 2,4-diterbutylphenol* |
| 10 | Pinocamphone | **Functionalized sesquiterpenoids** | |
| 11 | α -Terpineol | 27 | Longicamphenylone |
| 12 | Myrtenal | 28 | Caryophellene oxide |
| 13 | Estragol* | **Functionalized triterpenoids** | |
| 14 | Verbenone | 29 | Manoyl oxide |
| 15 | m-diterbutylbenzene | 30 | 13-epi-Manoyl oxide |
| 16 | Bornyl acetate | | |

Three families of compounds were identified: monoterpenoids, sesquiterpenoids and triterpenoids. Within these families of compounds are present aliphatic members and functionalized members with alcohol, aldehyde, ketone, ether, acetate and oxide functionalized groups.

The peak that can be seen in the chromatogram of figure 7 between 2 and 5 min is a mixture of the most volatile compounds with a high amount of CO₂, which mask other compounds, and also O₂.

A similar experiment was made with oak (Quercus petraea) and beech (Fagus sylvatica) wood cored. These experiments made possible to identify series of n-alkanes, branched n-alkanes, n-aldehydes and n-alcohols, including acetic acid in the most volatile fraction.

Two wood cores were introduced for 24 hours in 2l bag filled with the mentioned mixture of dry air as described for the experiment one done with the water standards according to figure 6 for vapour mode. As for the experiment one the vapour was passed through the multi-bed trap 10. Later the compounds trapped in the multi-bed trap were analysed in the TDU-GC-MS. In these samples, in the region of the most volatile compounds some low molecular weight ketones (C3-C5) and ethanol were identified.

Further analyses can be done to separate the most volatile fraction and identify all the trapped low molecular weight organic compounds. For this purpose, a cold trap can be incorporated between the TDU and the GC-MS.

In summary, the two experiments discussed in relation with figures 5 to 8 show that the multi-bed trap plays its expected role of trapping organic compounds while offering a reliable result for the isotope analysis.

The method and system of the invention are therefore particularly adapted for purifying water that is extracted or sublimated together with organic compounds from a wide range of matrixes with a high recovery yield and without modifying the initial isotope signature of the extracted/sublimated water. That being said, the method and system of the invention are not limited to this particular application. Additionally, the trapped organic compounds can be analysed if needed.

## Claims

1. System (1) for purifying water vapour samples prior to water stable isotope analysis, **characterized by**:
- a pipe (12) with a multi-bed trap (10) that comprises at least one hydrophobic graphitized black carbon sorbent, GBC (14, 16) and a hydrophobic carbon molecular sieve sorbent, CMS (18);
- an analyser (30) for analysing the isotope ratio of the purified water vapour; and
- a pump operatively connected to the pipe (12) and optionally constituting a part of the analyser, to lead water vapour through the at least one hydrophobic graphitized black carbon sorbent (14, 16) and then through the hydrophobic carbon molecular sieve sorbent (18).

2. System (1) according to claim 1, **characterized in that** the at least one hydrophobic graphitized black carbon sorbent (14, 16) comprises a first sorbent (14) and a second sorbent (16), the water vapour passing through the first sorbent (14) before passing through the second sorbent (16), the first sorbent (14) having a lower effective surface area than the second sorbent (16), preferably wherein the effective surface area of the first sorbent (14) is lower than 25 m²/g and/or the effective surface area of the second sorbent (16) is greater than 90 m²/g.

3. System (1) according to any of claims 1-2, **characterized in that** the effective surface area of the hydrophobic carbon molecular sieve sorbent (18) is greater than 800 m²/g.

4. System (1) according to any of claims 1-3, comprising a third hydrophobic graphitized carbon black sorbent placed upstream the first hydrophobic graphitized carbon black sorbent (14), the third hydrophobic graphitized carbon black sorbent having an effective surface area lower than the surface of the two subsequent hydrophobic graphitized carbon black sorbents (14, 16), preferably wherein the effective surface area of the third hydrophobic graphitized carbon black sorbent is lower than 10 m²/g.

5. System (1) according to any of claims 1-4, **characterized in that** it comprises at least one inert and hydrophobic frit filter (19, 20, 22, 21) interposed between the sorbents (14, 16, 18) and/or retaining the sorbents (14, 16, 18) in the pipe (12), preferably wherein the at least one inert and hydrophobic frit filter (19, 20, 22, 21) is an inert-coated stainless steel frit filter or a quartz frit filter or a glass frit filter, and preferably wherein the system (1) further comprises a hydrophobic retaining element (23) arranged downstream of, and to retain, the hydrophobic frit filter (21), the hydrophobic retaining element (23) preferably being formed by an inert-coated stainless steel wire cloth basket.

6. System (1) according to any of claims 1-5, **characterized in that** the pipe (12) has an internal diameter of about 4 mm; and/or the multi-bed trap (10) spans over about 60 mm of length of the pipe (12).

7. System (1) according to any of claims 1-6, further comprising a vapour source (24, 28).

8. System (1) according to claim 7, **characterized in that** the vapour source is a sublimation system (24) connected to the pipe (12) and forming water vapour by sublimation from a solid sample.

9. System (1) according to claim 7, **characterized in that** the vapour source is a cryo-extraction system; or a transfer system connected to the pipe (12) and forming water vapour by sublimation from a solid sample or by transfer from a collection system.

10. System (1) according to claim 7, **characterized in that** the vapour source is an inlet system (28) connected to the pipe (12), the inlet system (28) being configurated to directly introduce the sample in vapour phase (vapour mode) towards the pipe (12) or the inlet system (28) being a combination of injector and vaporizer (liquid mode) to vaporize the injected liquid water sample before being directed to the pipe (12).

11. System (1) according to any of claims 1-10, **characterized in that** the pipe (12) comprises at least one U-shape cold trap (26, 27) arranged upstream and/or downstream of the multi-bed trap (10), preferably wherein the analyser (30) is directly connected to the cold trap (26, 27) placed downstream the multi-bed trap (10).

12. System (1) according to any of claims 1-11, **characterized in that** the pipe (12) comprises at least one sample tube (32) arranged upstream and/or downstream of the multi-bed trap (10).

13. System (1) according to any of claims 1-12, **characterized in that** the analyser (30) is directly connected to the multi-bed trap (10).

14. System (1) according to any of claims 1-13, **characterized in that** the analyser (30) is an isotope-ratio infrared spectroscopy, IRIS, based instrument, i.e., a wavelength-scanned cavity ring-down spectroscopy analyser; or a mass spectrometer.

15. Method for purifying and analysing water vapour comprising:
- leading water vapour in a pipe (12) through at least one hydrophobic graphitized black carbon sorbent (14, 16) and then through a hydrophobic carbon molecular sieve sorbent (18); and then
- analysing the isotope ratio of the purified water vapour,
preferably wherein the method is carried out in a system (1) according to any of claims 1 to 14.

## Patentansprüche

1. System (1) zur Reinigung von Wasserdampfproben vor der Analyse stabiler Wasserisotope, **gekennzeichnet durch**:
- ein Rohr (12) mit einer Mehrschichtfalle (10), die mindestens einen hydrophoben graphitierten schwarzen Kohlenstoffsorbens, GBC (14, 16) und einen hydrophoben Kohlenstoff-Molekularsieb-Sorbens, CMS (18) umfasst;
- einen Analysator (30) zur Analyse des Isotopenverhältnisses des gereinigten Wasserdampfs; und
- eine Pumpe, die operativ mit dem Rohr (12) verbunden ist und optional einen Teil des Analysators bildet, um Wasserdampf durch den mindestens einen hydrophoben graphitierten schwarzen Kohlenstoffsorbens (14, 16) und dann durch den hydrophoben Kohlenstoff-Molekularsieb-Sorbens (18) zu führen.

2. System (1) nach Anspruch 1, **gekennzeichnet dadurch, dass** der mindestens eine hydrophobe graphitierte schwarze Kohlenstoffsorbens (14, 16) einen ersten Sorbens (14) und einen zweiten Sorbens (16) umfasst, wobei der Wasserdampf durch den ersten Sorbens (14) hindurchgeht, bevor er durch den zweiten Sorbens (16) hindurchgeht, wobei der erste Sorbens (14) eine geringere effektive Oberfläche als der zweite Sorbens (16) hat, vorzugsweise wobei die effektive Oberfläche des ersten Sorbens (14) kleiner als 25 m²/g ist und/oder die effektive Oberfläche des zweiten Sorbens (16) größer als 90 m²/g ist.

3. System (1) nach einem der Ansprüche 1-2, **gekennzeichnet dadurch, dass** die effektive Oberfläche des hydrophoben Kohlenstoff-Molekularsieb-Sorbens (18) größer als 800 m²/g ist.

4. System (1) nach einem der Ansprüche 1-3, umfassend einen dritten hydrophoben graphitierten schwarzen Kohlenstoffsorbens, der stromaufwärts des ersten hydrophoben graphitierten schwarzen Kohlenstoffsorbens (14) platziert ist, wobei der dritte hydrophobe graphitierte schwarze Kohlenstoffsorbens eine geringere effektive Oberfläche als die Oberfläche der beiden nachfolgenden hydrophoben graphitierten schwarzen Kohlenstoffsorbens (14, 16) hat, vorzugsweise wobei die effektive Oberfläche des dritten hydrophoben graphitierten schwarzen Kohlenstoffsorbens kleiner als 10 m²/g ist.

5. System (1) nach einem der Ansprüche 1-4, **gekennzeichnet dadurch, dass** es mindestens einen inert und hydrophoben Frittenfilter (19, 20, 22, 21) umfasst, der zwischen den Sorbenten (14, 16, 18) eingefügt ist und/oder die Sorbenten (14, 16, 18) im Rohr (12) zurückhält, vorzugsweise wobei der mindestens eine inert und hydrophobe Frittenfilter (19, 20, 22, 21) ein inert beschichteter Edelstahl-Frittenfilter oder ein Quarz-Frittenfilter oder ein Glas-Frittenfilter ist, und vorzugsweise wobei das System (1) ferner ein hydrophobes Rückhalteelement (23) umfasst, das stromabwärts des hydrophoben Frittenfilters (21) angeordnet ist und diesen zurückhält, wobei das hydrophobe Rückhalteelement (23) vorzugsweise durch einen inert beschichteten Edelstahl-Drahtgeflechtkorb gebildet wird.

6. System (1) nach einem der Ansprüche 1-5, **gekennzeichnet dadurch, dass** das Rohr (12) einen Innendurchmesser von etwa 4 mm hat; und/oder die Mehrschichtfalle (10) sich über etwa 60 mm Länge des Rohrs (12) erstreckt.

7. System (1) nach einem der Ansprüche 1-6, ferner umfassend einen Dampferzeuger (24, 28).

8. System (1) nach Anspruch 7, **gekennzeichnet dadurch, dass** der Dampferzeuger ein Sublimationssystem (24) ist, das mit dem Rohr (12) verbunden ist und Wasserdampf durch Sublimation aus einer festen Probe bildet.

9. System (1) nach Anspruch 7, **gekennzeichnet dadurch, dass** der Dampferzeuger ein Kryo-Extraktionssystem ist; oder ein Transfersystem, das mit dem Rohr (12) verbunden ist und Wasserdampf durch Sublimation aus einer festen Probe oder durch Transfer aus einem Sammelsystem bildet.

10. System (1) nach Anspruch 7, **gekennzeichnet dadurch, dass** der Dampferzeuger ein Einlasssystem (28) ist, das mit dem Rohr (12) verbunden ist, wobei das Einlasssystem (28) so konfiguriert ist, dass es die Probe direkt in der Dampfphase (Dampfmodus) in Richtung des Rohrs (12) einführt oder das Einlasssystem (28) eine Kombination aus Injektor und Verdampfer (Flüssigkeitsmodus) ist, um die injizierte flüssige Wasserprobe zu verdampfen, bevor sie in Richtung des Rohrs (12) geleitet wird.

11. System (1) nach einem der Ansprüche 1-10, **gekennzeichnet dadurch, dass** das Rohr (12) mindestens eine U-förmige Kaltfalle (26, 27) umfasst, die stromaufwärts und/oder stromabwärts der Mehrschichtfalle (10) angeordnet ist, vorzugsweise wobei der Analysator (30) direkt mit der stromabwärts der Mehrschichtfalle (10) platzierten Kaltfalle (26, 27) verbunden ist.

12. System (1) nach einem der Ansprüche 1-11, **gekennzeichnet dadurch, dass** das Rohr (12) mindestens ein Probenrohr (32) umfasst, das stromaufwärts und/oder stromabwärts der Mehrschichtfalle (10) angeordnet ist.

13. System (1) nach einem der Ansprüche 1-12, **gekennzeichnet dadurch, dass** der Analysator (30) direkt mit der Mehrschichtfalle (10) verbunden ist.

14. System (1) nach einem der Ansprüche 1-13, **gekennzeichnet dadurch, dass** der Analysator (30) ein auf Isotopenverhältnis-Infrarotspektroskopie, IRIS, basierendes Instrument ist, d.h. ein Wellenlängen-gescanntes Hohlraum-Ring-Down-Spektroskopie-Analysator; oder ein Massenspektrometer.

15. Verfahren zur Reinigung und Analyse von Wasserdampf, umfassend:
- das Führen von Wasserdampf in einem Rohr (12) durch mindestens einen hydrophoben graphitierten schwarzen Kohlenstoffsorbens (14, 16) und dann durch einen hydrophoben Kohlenstoff-Molekularsieb-Sorbens (18); und dann
- die Analyse des Isotopenverhältnisses des gereinigten Wasserdampfs,
vorzugsweise wobei das Verfahren in einem System (1) nach einem der Ansprüche 1 bis 14 durchgeführt wird.

## Revendications

1. Système (1) pour purifier des échantillons de vapeur d'eau avant l'analyse des isotopes stables de l'eau, **caractérisé par** :
- un tuyau (12) avec un piège multi-couches (10) qui comprend au moins un adsorbant hydrophobe de carbone noir graphité, CNG (14, 16) et un adsorbant hydrophobe de tamis moléculaire de carbone, TMC (18) ;
- un analyseur (30) pour analyser le rapport isotopique de la vapeur d'eau purifiée ; et
- une pompe connectée de manière opérative au tuyau (12) et constituant éventuellement une partie de l'analyseur, pour conduire la vapeur d'eau à travers au moins un adsorbant hydrophobe de carbone noir graphité (14, 16) puis à travers l'adsorbant hydrophobe de tamis moléculaire de carbone (18).

2. Système (1) selon la revendication 1, **caractérisé en ce que** le ou les adsorbants hydrophobes de carbone noir graphité (14, 16) comprennent un premier adsorbant (14) et un deuxième adsorbant (16), la vapeur d'eau passant à travers le premier adsorbant (14) avant de passer à travers le deuxième adsorbant (16), le premier adsorbant (14) ayant une surface effective inférieure à celle du deuxième adsorbant (16), de préférence où la surface effective du premier adsorbant (14) est inférieure à 25 m²/g et/ou la surface effective du deuxième adsorbant (16) est supérieure à 90 m²/g.

3. Système (1) selon l'une quelconque des revendications 1-2, **caractérisé en ce que** la surface effective de l'adsorbant hydrophobe de tamis moléculaire de carbone (18) est supérieure à 800 m²/g.

4. Système (1) selon l'une quelconque des revendications 1-3, comprenant un troisième adsorbant hydrophobe de carbone noir graphité placé en amont du premier adsorbant hydrophobe de carbone noir graphité (14), le troisième adsorbant hydrophobe de carbone noir graphité ayant une surface effective inférieure à celle des deux adsorbants hydrophobes de carbone noir graphité suivants (14, 16), de préférence où la surface effective du troisième adsorbant hydrophobe de carbone noir graphité est inférieure à 10 m²/g.

5. Système (1) selon l'une quelconque des revendications 1-4, **caractérisé en ce qu'**il comprend au moins un filtre frit hydrophobe et inerte (19, 20, 22, 21) interposé entre les adsorbants (14, 16, 18) et/ou retenant les adsorbants (14, 16, 18) dans le tuyau (12), de préférence où le ou les filtres frits hydrophobes et inertes (19, 20, 22, 21) sont des filtres frits en acier inoxydable revêtu d'inertie ou des filtres frits en quartz ou des filtres frits en verre, et de préférence où le système (1) comprend en outre un élément de retenue hydrophobe (23) disposé en aval du filtre frit hydrophobe (21) et destiné à retenir ce dernier, l'élément de retenue hydrophobe (23) étant de préférence formé par un panier en toile métallique en acier inoxydable revêtu d'inertie.

6. Système (1) selon l'une quelconque des revendications 1-5, **caractérisé en ce que** le tuyau (12) a un diamètre interne d'environ 4 mm ; et/ou le piège multi-couches (10) s'étend sur environ 60 mm de longueur du tuyau (12).

7. Système (1) selon l'une quelconque des revendications 1-6, comprenant en outre une source de vapeur (24, 28).

8. Système (1) selon la revendication 7, **caractérisé en ce que** la source de vapeur est un système de sublimation (24) connecté au tuyau (12) et formant de la vapeur d'eau par sublimation à partir d'un échantillon solide.

9. Système (1) selon la revendication 7, **caractérisé en ce que** la source de vapeur est un système de cryo-extraction ; ou un système de transfert connecté au tuyau (12) et formant de la vapeur d'eau par sublimation à partir d'un échantillon solide ou par transfert à partir d'un système de collecte.

10. Système (1) selon la revendication 7, **caractérisé en ce que** la source de vapeur est un système d'entrée (28) connecté au tuyau (12), le système d'entrée (28) étant configuré pour introduire directement l'échantillon en phase vapeur (mode vapeur) vers le tuyau (12) ou le système d'entrée (28) étant une combinaison d'injecteur et de vaporisateur (mode liquide) pour vaporiser l'échantillon d'eau liquide injecté avant d'être dirigé vers le tuyau (12).

11. Système (1) selon l'une quelconque des revendications 1-10, **caractérisé en ce que** le tuyau (12) comprend au moins un piège froid en forme de U (26, 27) disposé en amont et/ou en aval du piège multi-couches (10), de préférence où l'analyseur (30) est directement connecté au piège froid (26, 27) placé en aval du piège multi-couches (10).

12. Système (1) selon l'une quelconque des revendications 1-11, **caractérisé en ce que** le tuyau (12) comprend au moins un tube d'échantillon (32) disposé en amont et/ou en aval du piège multi-couches (10).

13. Système (1) selon l'une quelconque des revendications 1-12, **caractérisé en ce que** l'analyseur (30) est directement connecté au piège multi-couches (10).

14. Système (1) selon l'une quelconque des revendications 1-13, **caractérisé en ce que** l'analyseur (30) est un instrument basé sur la spectroscopie infrarouge à rapport isotopique, IRIS, c'est-à-dire un analyseur de spectroscopie par décroissance de cavité balayée en longueur d'onde ; ou un spectromètre de masse.

15. Procédé de purification et d'analyse de la vapeur d'eau comprenant :
- conduire la vapeur d'eau dans un tuyau (12) à travers au moins un adsorbant hydrophobe de carbone noir graphité (14, 16) puis à travers un adsorbant hydrophobe de tamis moléculaire de carbone (18) ; et ensuite
- analyser le rapport isotopique de la vapeur d'eau purifiée,
de préférence où le procédé est réalisé dans un système (1) selon l'une quelconque des revendications 1 à 14.
